(19) **Europäisches Patentamt / European Patent Office / Office européen des brevets**

(11) **EP 3 603 563 A1**

(12) **EUROPEAN PATENT APPLICATION**
published in accordance with Art. 153(4) EPC

(43) Date of publication:
**05.02.2020 Bulletin 2020/06**

(21) Application number: **18776265.3**

(22) Date of filing: **28.03.2018**

(51) Int Cl.:
*A61B 90/90* [(2016.01)]   *A61B 17/28* [(2006.01)]
*A61B 17/30* [(2006.01)]   *A61B 17/3211* [(2006.01)]

(86) International application number:
**PCT/JP2018/012869**

(87) International publication number:
**WO 2018/181526 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2017 JP 2017070408**
**13.12.2017 JP 2017238203**

(71) Applicant: **Daicel Polymer Ltd.**
**Tokyo 108-8231 (JP)**

(72) Inventors:
- **ITAKURA Masahiko**
  **Tokyo 108-8231 (JP)**
- **KATAYAMA Masahiro**
  **Himeji-shi**
  **Hyogo 671-1123 (JP)**
- **UNO Takayuki**
  **Himeji-shi**
  **Hyogo 671-1123 (JP)**

(74) Representative: **Grünecker Patent- und Rechtsanwälte**
**PartG mbB**
**Leopoldstraße 4**
**80802 München (DE)**

(54) **MEDICAL APPARATUS AND METHOD FOR MANUFACTURING SAME**

(57)   The present invention provides a medical apparatus with an IC tag and a method for manufacturing the same. Provided are a method for manufacturing a medical apparatus and a medical apparatus manufactured by the same, the method including: irradiating a portion of a surface of a medical apparatus made of a metal with a laser beam to roughen the surface and form a roughened section; depositing a synthetic resin in a molten state or a solution state to the portion of the medical apparatus including the roughened section to form a base section of synthetic resin; placing an IC tag on the base section; and depositing a synthetic resin in a molten state or a solution state onto the base section and the IC tag to form a covering section of the synthetic resin that covers the base section of the synthetic resin and the IC tag, thereby encapsulating the IC tag inside a synthetic resin part including the base section and the covering section.

FIG. 4A

# FIG. 4B

21 ⌒      31      20

# FIG. 4C

40

21      31      20

# FIG. 4D

30      20

21

**Description**

Technical Field

The present invention relates to a medical apparatus such as a scalpel, scissors, forceps, and tweezers, and to a manufacturing method thereof.

Background Art

The usage history of medical apparatuses for use in medical procedures such as surgery is precisely managed for each individual medical apparatus, and these medical apparatuses are individually managed to prevent their loss. Examples of such medical apparatuses include surgical equipment and materials such as scalpels, scissors, forceps and tweezers.

As a method to ensure and simplify such management, WO 2014/017530 proposes an invention of a medical apparatus with an IC tag affixed and a method for affixing the same. WO 2014/017530 describes a method of attaching an IC tag to a medical apparatus using a cover body and an adhesive.

Summary of Invention

An object of the present invention is to provide a medical apparatus having an IC tag attached with a very strong adhesive force, and a method for manufacturing the same.

The present invention provides a medical apparatus made of a metal with an IC tag encapsulated, the medical apparatus including: a synthetic resin part affixed to a roughened section of the medical apparatus made of a metal, the roughened section having a porous structure including holes with a depth from 10 to 900 $\mu$m; and the IC tag encapsulated inside the synthetic resin part.

The present invention also provides a method for manufacturing the above-mentioned medical apparatus, the method including:

(A) irradiating a portion of a surface of a medical apparatus made of a metal with a laser beam to roughen the surface and form a roughened section;
(B) depositing a synthetic resin in a molten state or a solution state onto the portion of the medical apparatus including the roughened section to form a base section of the synthetic resin;
(C) placing an IC tag on the base section; and
(D) depositing a synthetic resin in a molten state or a solution state onto the base section and the IC tag to form a covering section of the synthetic resin that covers the base section of the synthetic resin and the IC tag, thereby encapsulating the IC tag inside a synthetic resin part including the base section and the covering section.

The IC tag of the medical apparatus of the present invention is attached with a strong adhesive force in a state in which the IC tag is covered with a synthetic resin, and therefore the IC tag does not detach even in cases in which a repeatedly used medical apparatus is repeatedly cleaned and sterilized.

As such, the usage history of the medical apparatus can be managed for an extended period of time, and a significant effect of preventing loss of the medical apparatus is provided.

Brief Description of Drawings

FIG. 1 is a plan view of a medical apparatus (forceps) of an embodiment of the present invention.
FIG. 2 is a side view of a medical apparatus (tweezers) of an embodiment of the present invention.
FIG. 3 is a partial enlarged cross-sectional view of the medical apparatus illustrated in FIG. 1 or FIG. 2.
FIG. 4 is a diagram for describing a method for manufacturing the medical apparatus illustrated in FIG. 2.
FIG. 5 is an SEM photograph (40 times) of a titanium alloy surface after irradiation with a laser beam in Example 6.
FIG. 6 is an SEM photograph (40 times) of a titanium alloy surface after irradiation with a laser beam in Example 7.
FIG. 7 is an SEM photograph (40 times) of a titanium alloy surface after irradiation with a laser beam in Example 8.

Description of Embodiments

Medical Apparatus

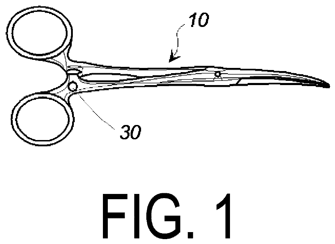

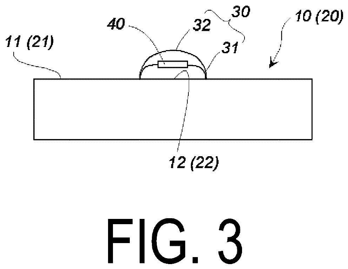

A medical apparatus made of a metal illustrated in FIG. 1 is a forceps 10, which, as illustrated in FIG. 3, includes: a roughened section 12 having a porous structure including numerous holes with a depth from 10 to 900 $\mu$m formed in a portion of a surface 11 of the forceps 10, a synthetic resin part 30 affixed to the roughened section 12, and an IC tag 40 encapsulated inside the synthetic resin part 30. Here, "hole" is a concept that includes a groove.

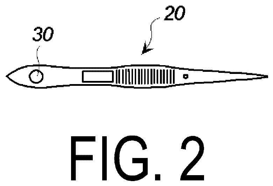

A medical apparatus made of a metal illustrated in FIG. 2 is a tweezers 20, which, as illustrated in FIG. 3, includes: a roughened section 22 having a porous structure including numerous holes with a depth from 10 to 900 $\mu$m formed in a portion of a surface 21 of the tweezers 20, a synthetic resin part 30 affixed to the roughened section 22, and an IC tag 40 encapsulated inside the synthetic resin part 30.

The synthetic resin part 30 includes a base section (lower layer part) 31 and a covering section (upper layer part) 32. The base section 31 and the covering section 32 are integrated, and the IC tag 40 is encapsulated therein.

The synthetic resin is selected from known thermoplastic resins, thermosetting resins, and energy ray curable resins and the like, and may be a synthetic resin adhesive or a rubber-based adhesive.

Furthermore, the base section 31 and the covering section 32 may be made from the same synthetic resin, or may be made from different synthetic resins. For example, the base section 31 may be made from a thermoplastic resin, and the covering section 32 may be made from an energy ray curable resin. The thickness of the synthetic resin part 30 from the surface 11 (surface 21) is preferably from 2 to 20 mm.

Method for Manufacturing the Medical Apparatus

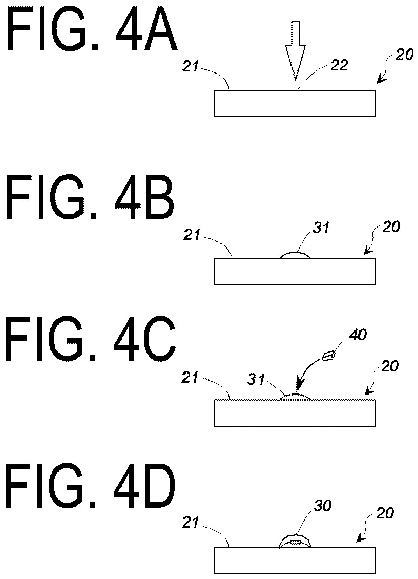

A method for manufacturing the medical apparatus of an embodiment of the present invention is described with reference to FIG. 4 for each step. The medical apparatus is described for the case of the tweezers 20 illustrated in FIG. 2.

Step Illustrated in FIG. 4A

In the initial step, as illustrated in FIG. 4A, a portion of the surface 21 of the tweezers 20 is roughened by performing irradiation with a laser beam, to form a roughened section 22 having a porous structure including numerous holes (surface roughening).

The roughened section 12 is favorably a portion of the surface of the tweezers 20 that does not come into contact with the affected area during surgery, and a region that does not come into contact with the hand at the base portion, or the like, is preferable.

The area of the roughened section 12 needs only be greater than the area of the IC tag 40, and for example, may be approximately from 10 to 100 mm$^2$.

As a method for roughening the surface by performing irradiation with a laser beam, a method of performing irradiation with a continuous wave laser beam or a pulsed wave laser beam can be used.

The method for performing irradiation with a continuous wave laser beam can be implemented in the same manner as methods for continuous irradiation with a laser beam described in JP 5774246 B, JP 5701414 B, JP 5860190 B, JP 5890054 B, JP 5959689 B, JP 2016-43413 A, JP 2016-36884 A, and JP 2016-44337 A.

When a continuous wave laser beam is used, a method of performing continuous irradiation with a laser beam having an energy density of 1 MW/cm$^2$ or greater at an irradiation rate of 2000 mm/sec or greater using a laser device is preferable.

The energy density (W/$\mu$m$^2$) for irradiation with a laser beam is determined from the laser output power (W) and the laser irradiation spot area ($\pi \times$ [(spot diameter)/2]$^2$). The energy density for irradiation with a laser beam is preferably from 2 to 1000 MW/cm$^2$, more preferably from 10 to 800 MW/cm$^2$, and even more preferably from 10 to 700 MW/cm$^2$.

The irradiation rate of the laser beam is more preferably from 2000 to 20000 mm/sec, even more preferably from 2000 to 18000 mm/sec, and yet even more preferably from 3000 to 15000 mm/sec.

The output power of the laser beam is preferably from 4 to 4000 W, more preferably from 50 to 2500 W, and even more preferably from 150 to 2000 W. In a case where the other irradiation conditions of laser beam are the same, as the output power increases, the depth of the holes (grooves) becomes deeper, and as the output power decreases, the depth of the holes (grooves) becomes shallower.

The wavelength of the laser beam is preferably from 500 to 11000 nm.

The beam diameter (spot diameter) of the laser beam is preferably from 5 to 80 $\mu$m.

The defocus distance of the laser beam is preferably from -5 to +5 mm, more preferably from -1 to +1 mm, and even more preferably from -0.5 to +0.1 mm. Laser irradiation may be performed with the defocus distance set to a constant value, or may be performed while changing the defocus distance. For example, when laser irradiation is performed, the defocus distance may be set to be reduced, or may be set to periodically increase and decrease. In a case where the defocus distance is negative at an appropriate value, the depth of the holes formed will become deeper.

Also, the depth of the holes can be adjusted by adjusting the number of repetitions of irradiation with the laser beam. The number of repetitions (the total number of times that irradiation with the laser beam is performed to form a single hole or groove) is preferably from 1 to 9 and more preferably from 2 to 5. In a case where the same laser irradiation conditions are used, as the number of repetitions increases, the depth of the hole (groove) becomes deeper, and as the number of repetitions is reduced, the depth of the hole (groove) becomes shallower.

In addition to ordinary methods of irradiation with a pulsed wave laser beam, the method of irradiation with a pulsed wave laser beam can be performed in the same manner as the methods for irradiation with a pulsed wave laser beam described in JP 5848104 B, JP 5788836 B, JP 5798534 B, JP 5798535 B, and JP 2016-203643 A.

As the method for irradiation with a pulsed wave laser beam, a method of irradiation with a pulsed wave laser beam can be used with adjustment of one or more conditions selected from the following conditions (a) to (f).

Condition(a): The irradiation direction and irradiation angle when irradiating the medical apparatus made of a metal with the laser beam

Fixing of the irradiation direction of the laser beam to a specific direction and a specific angle can impart an orientation to the holes formed.

In addition, a method of irradiating a surface that includes the surface layer portion of the medical apparatus made of a metal with a laser beam in a direction perpendicular thereto is combined with a method of irradiating the surface that includes the surface layer portion of the medical apparatus made of a metal with a laser beam at an angle from 15 degrees to 85 degrees, to irradiate the surface with the laser beam at different angles, thereby controlling the size, shape, and depth of the holes.

Condition (b): The irradiation rate when irradiating the medical apparatus made of a metal with the laser beam

The irradiation rate of the laser beam is preferably from 1 to 10000 mm/sec and more preferably from 10 to 1000 mm/sec.

Condition (c): The energy density when irradiating the medical apparatus made of a metal with the laser beam

The energy density is preferably 0.3 GW/cm$^2$ or greater. The energy density for irradiation with the laser beam is determined from the output power (W) of the laser beam and the spot area (cm$^2$) ($\pi\square[$(spot diameter)$/2]^2$) of the laser beam. The energy density at the time of irradiation with the laser beam is more preferably from 0.3 to 1000 GW/cm$^2$, even more preferably from 1 to 800 GW/cm$^2$, and yet even more preferably from 1 to 500 GW/cm$^2$. As the energy density is increased, the holes become deeper and larger.

The output power of the laser beam is preferably from 4 to 400 W, more preferably from 5 to 100 W, and even more preferably from 10 to 100 W. In a case where the other irradiation conditions of the laser beam are the same, as the output power increases, the holes become deeper and larger, and as the output power decreases, the holes become shallower and smaller.

Condition (d): Number of repetitions of irradiation with the laser beam

The number of repetitions (the total number of times that irradiation with the laser beam is performed to form a single hole) is preferably from 1 to 200, and more preferably from 3 to 100. In a case where the same laser irradiation conditions are used, as the number of repetitions increases, the holes become deeper and larger, and as the number of repetitions is reduced, the holes become shallower and smaller.

Condition (e): The irradiation form when irradiating the medical apparatus made of a metal with the laser beam

The irradiation form includes: (e-1) a form in which the laser beam is irradiated in a state in which the medical apparatus made of a metal is in contact with a shaped body having a thermal conductivity that differs from that of the metal constituting the medical apparatus made of a metal, or

(e-2) a form in which the medical apparatus made of a metal is irradiated with a laser beam while the medical apparatus made of a metal is held in midair.

A method of (i) or (ii) below can be applied as the irradiation form of the condition (e-1).

(i) A method of irradiation with a laser beam in a state in which a surface of the medical apparatus made of a metal that is not to be irradiated by the laser beam is in contact with a substrate (for example, a copper plate or an aluminum plate) made from a material having a greater thermal conductivity (for example, a material having a thermal conductivity of 100 W/m□k or greater) than that of the metal constituting the medical apparatus made of a metal. As the method of (i), the method described in JP 2016-78090 A can be applied.

(ii) A method of irradiation with a laser beam in a state in which a surface of the medical apparatus made of a metal that is not to be irradiated by the laser beam is in contact with a substrate (for example, a glass plate) made from a material having a smaller thermal conductivity than that of the metal constituting the medical apparatus made of a metal. As the method of (ii), the method described in JP 2016-124024 A can be applied.

The method of (i) can suppress an increase in temperature by dissipating the heat that is generated when irradiating the medical apparatus made of a metal with the laser beam. The method of (ii) can suppress the dissipation of heat that is generated when irradiating the medical apparatus made of a metal with the laser beam.

Therefore, when the method of (i) is implemented, changes in the size, depth, and shape of the holes can be suppressed, and when the method of (ii) is implemented, changes in the size, depth, and shape of the holes can be facilitated. Thus, the size, depth, and shape of the holes can be adjusted by using the method of (i) and the method of (ii) for different purposes.

The irradiation form of the condition (e-2) is a form in which the medical apparatus made of a metal is irradiated with a laser beam while being held in midair with a holding means such as a clamp. The dissipation of heat that is generated when irradiating the metal medical apparatus with the laser beam can be suppressed by holding the metal medical apparatus in midair.

Furthermore, as another condition (e-3) of the irradiation form of the condition (e), irradiation with the laser beam can be performed under supplying an assist gas selected from air, oxygen, nitrogen, and argon, and additionally, irradiation with the laser beam can also be performed in a vacuum atmosphere (reduced pressure atmosphere).

With the irradiation form of the condition (e-3), the type of assist gas and the supply pressure (MPa) of the gas are preferably adjusted. Irradiation with the laser beam under supplying the assist gas assists in controlling the depth, size, and orientation of the holes, and also makes it possible to suppress the production of carbonized products and control surface properties.

For example, when argon gas is selected, oxidation of the surface can be prevented, when oxygen gas is selected, oxidation of the surface can be promoted, and when nitrogen gas is selected, surface hardness can be improved.

(f) The interval between lines or dots when irradiating the medical apparatus made of a metal with the laser beam

When the medical apparatus made of a metal is irradiated with a laser beam in a line shape, the interval between adjacent lines can be made wider or narrower, and thereby the size of the holes, the shape of the holes, and the depth of the holes can be adjusted.

Note that with a pulsed wave laser beam, the laser beam cannot be irradiated in a continuous straight line like a continuous wave laser beam. Instead, the laser beam is irradiated in dots, a plurality of these dots being connected to form a line; or alternatively, the pulsed wave laser beam can also be irradiated in a manner that a large number of dots are formed at intervals without forming a line.

The interval between lines or the interval between dots is preferably in a range from 0.01 to 1 mm.

A narrow interval between lines or a narrow interval between dots has a thermal impact on adjacent lines or adjacent dots, and therefore the holes become large, the shape of the holes becomes more complex, and the depth of the holes tends to become deeper, and in a case where the thermal impact is too great, a proper hole shape may not be formed.

When the interval between lines or the interval between dots is wide, the holes become smaller, the shape of the holes does not become complex, and the hole does not tend to be very deep, but the processing speed can be increased.

In addition, the wavelength of the pulsed wave laser beam is preferably from 500 to 11000 nm, and the beam diameter (spot diameter) of the pulsed wave laser beam is preferably from 5 to 80 μm. The frequency of the pulsed wave laser beam is preferably from 1 to 100 kHz, and the pulse width is preferably from 1 to 10000 nsec.

In a preferred embodiment of the surface roughening step, a fiber laser device in which a direct modulation type modulation device that directly converts the laser drive current is connected to the laser power supply can be used to adjust the duty ratio for laser irradiation.

There are two types of laser excitation: pulsed excitation and continuous excitation, and pulsed wave lasers that are pulsed through pulsed excitation are commonly referred to as normal pulses.

A pulsed wave laser can be produced even with continuous excitation. The pulsed wave laser can be produced by: a Q-switched pulse oscillation method that is capable of shortening the pulse width (pulse ON time) relative to a normal pulse, thereby oscillating a laser having a higher peak power; an external modulation system that generates a pulsed wave laser by extracting light in time domain using an AOM or LN light intensity modulator; a method of pulsing the laser beam by mechanical chopping; a method of pulsing the laser beam by operating a Galvano controller; and a direct modulation system that directly modulates the laser drive current to produce a pulsed wave laser.

Among these methods, the method of pulsing the laser beam by mechanical chopping, the method of pulsing the laser beam by operating a Galvano controller, and the direct modulation system that directly modulates the laser drive current to produce a pulsed wave laser are preferable because pulsing (irradiation such that irradiated and non-irradiated portions are alternately produced) can be easily performed without changing the energy density of the continuous wave laser.

In one preferred embodiment of the present invention, a fiber laser device in which a direct modulation type modulation device that directly converts the laser drive current is connected to the laser power supply is used to continuously excite the laser and produce a pulsed wave laser.

The duty ratio is a ratio determined by the following equation from the ON time and OFF time of the laser beam output.

$$\text{Duty Ratio (\%)} = (\text{ON time})/(\text{ON time} + \text{OFF time}) \times 100$$

The duty ratio corresponds to L1/(L1 + L2) where the length of the portion irradiated by the laser beam is L1 and the length of the portion not irradiated by the laser beam is L2, and the duty ratio can be selected from a range from 10 to 90%.

The laser beam can be irradiated in a dotted line by adjusting the duty ratio and performing irradiation with the laser beam. When the duty ratio is large, the efficiency of the surface roughening step improves, but the cooling effect deteriorates, and when the duty ratio is small, the cooling effect improves, but the surface roughening efficiency becomes poor. The duty ratio is preferably adjusted according to the purpose.

The length (L1) of the portion irradiated by the laser beam and the length (L2) of the portion not irradiated by the laser beam can be adjusted to be in a range from L1/L2 = 1/9 to 9/1.

The length (L1) of the portion irradiated by the laser beam is preferably 0.05 mm or greater and more preferably from 0.1 to 1 mm in order to roughen the surface into a complex porous structure while manifesting a cooling effect.

A known laser can be used as the laser that is used in the irradiation of the laser beam, and for example, a $YVO_4$ laser, a fiber laser (single-mode fiber laser and multi-mode fiber laser), an excimer laser, a carbon dioxide laser, a UV laser, a YAG laser, a semiconductor laser, a glass laser, a ruby laser, a He-Ne laser, a nitrogen laser, a chelate laser, or a dye laser can be used.

The roughened section 22 of the tweezers 20 roughened in the step illustrated in FIG. 4A is in a state in which a large number of holes are formed (porous structure). The depth the holes from the surface 21 (the non-roughened surface of the tweezers 20) of the tweezers 20 to the bottom of the holes is preferably in a range from 10 to 900 $\mu$m. The depth of the holes is more preferably in a range from 20 to 500 $\mu$m and even more preferably in a range from 30 to 300 $\mu$m.

Step Illustrated in FIG. 4B

In the next step, a synthetic resin in a molten state or a solution state is deposited onto the portion of the tweezers 20 including the roughened section 22 to form the base section 31 of the synthetic resin part 30.

Since the base section 31 is for placing the IC tag 40 on the base section 31 in the next step illustrated in FIG. 4C, the base section 31 is preferably formed to have a wider area than the region occupied by the IC tag 40.

The synthetic resin may be in a form such that a small amount of synthetic resin can be deposited onto the roughened section 22, and a synthetic resin in a heating molten state or a synthetic resin in a solution state obtained by dissolving the synthetic resin in a solvent can be used. As the depositing method, a method in which a small amount of the synthetic resin is coated (dribbled) onto the roughened section 22 can be used, or potting or the like can be used.

Examples of the synthetic resin used in this step include thermoplastic resins, thermosetting resins, and thermoplastic elastomers, and energy ray curable resins can also be used. Synthetic resin adhesives and rubber-based adhesives can also be used as the synthetic resin.

In the step illustrated in FIG. 4B, depending on the type of the synthetic resin forming the base section 31, drying, heating, irradiation with energy beams, and the like can be performed, and then the synthetic resin can be cured to an extent such that when the IC tag 40 is stably placed on the base section 31 in the next step (cured to an extent that the synthetic resin is slightly recessed which allows the IC tag 40 to be stably placed when the IC tag 40 is placed thereon).

Note that the step illustrated in FIG. 4B may be omitted in consideration of the type, size, and shape of the medical apparatus to be adopted, and the size of the IC tag 40, or the base section 31 may be formed such that the area thereof is smaller than the area (occupied region) of the IC tag 40.

The thermoplastic resin can be appropriately selected from known thermoplastic resins according to the application. Examples include, but are not limited to, polyamide resins (aliphatic polyamides such as PA6 and PA66, and aromatic polyamides), polystyrene, copolymers containing styrene units such as ABS resin or AS resin, polyethylene, copolymers containing ethylene units, polypropylene, copolymers containing propylene units, other polyolefins, polyvinyl chloride, polyvinylidene chloride, polycarbonate resins, acrylic resins, methacrylic resins, polyester resins, polyacetal resins, and polyphenylene sulfide resins.

The thermosetting resin can be appropriately selected from known thermosetting resins according to the application. Examples include, but are not limited to, urea resins, melamine resins, phenolic resins, resorcinol resins, epoxy resins, polyurethanes, and vinyl urethanes.

The thermoplastic elastomer can be appropriately selected from known thermoplastic elastomers according to the application. Examples thereof include, but are not limited to, styrene-based elastomers, vinyl chloride-based elastomers, olefin-based elastomers, urethane-based elastomers, polyester-based elastomers, nitrile-based elastomers, and polyamide-based elastomers.

The energy ray curable resin is preferably selected from ultraviolet light curable resins and electron beam curable resins.

The energy ray curable resin is preferably selected from radically polymerizable monomers, oligomers of radically polymerizable monomers, cationically polymerizable monomers, and oligomers of cationically polymerizable monomers. The monomer or the oligomer that is in a liquid state (including a gel with a low viscosity) at normal temperature or that is in a solution form obtained by dissolving it in a solvent can be used as is, and a solid (powder) of the monomer or the oligomer can be used after heating melted or dissolved in a solvent.

Radically Polymerizable Monomer

Examples of radically polymerizable compounds include compounds having, per molecule, one or more radically polymerizable groups, such as (meth)acryloyl groups, (meth)acryloyloxy groups, (meth)acryloyl amino groups, vinyl ether groups, vinyl aryl groups, and vinyloxycarbonyl groups.

Examples of compounds having one or more (meth)acryloyl groups per molecule include, but are not limited to, 1-buten-3-one, 1-penten-3-one, 1-hexen-3-one, 4-phenyl-1-buten-3-one, 5-phenyl-1-penten-3-one, and derivatives thereof.

Compounds having one or more (meth)acryloyloxy group per molecule include, but are not limited to, methyl (meth)acrylate, ethyl (meth)acrylate, n-butyl (meth)acrylate, isobutyl (meth)acrylate, t-butyl (meth)acrylate, n-hexyl (meth)acrylate, 2-ethylhexyl (meth)acrylate, isodecyl (meth)acrylate, n-lauryl (meth)acrylate, n-stearyl (meth)acrylate, n-butoxyethyl (meth)acrylate, butoxy diethylene glycol (meth)acrylate, methoxy triethylene glycol (meth)acrylate, methoxy polyethylene glycol (meth)acrylate, cyclohexyl (meth)acrylate, tetrahydrofurfuryl (meth)acrylate, benzyl (meth)acrylate, phenoxyethyl (meth)acrylate, isobornyl (meth)acrylate, 2-hydroxyethyl (meth)acrylate, 2-hydroxypropyl (meth)acrylate, 2-hydroxybutyl (meth)acrylate, dimethylaminoethyl (meth)acrylate, diethylaminoethyl (meth)acrylate, acrylic acid, methacrylic acid, 2-(meth)acryloyloxyethyl succinate, 2-(meth)acryloyloxyethyl hexahydrophthalic acid, 2-(meth)acryloyloxyethyl-2-hydroxypropyl phthalate, glycidyl (meth)acrylate, 2-(meth)acryloyloxyethyl acid phosphate, ethylene glycol di(meth)acrylate, diethylene glycol di(meth)acrylate, triethylene glycol di(meth)acrylate, 1,4-butanediol di(meth)acrylate, neopentyl glycol di(meth)acrylate, 1,6-hexanediol di(meth)acrylate, 1,9-nonanediol di(meth)acrylate, 1,10-decanediol di(meth)acrylate, decane di(meth) acrylate, glycerin di(meth) acrylate, 2-hydroxy-3-(meth)acryloyloxypropyl (meth)acrylate, dimethylol tricyclodecane di(meth)acrylate, trifluoroethyl (meth)acrylate, perfluorooctyl ethyl (meth)acrylate, isoamyl (meth)acrylate, isomyristyl (meth)acrylate, γ-(meth)acryloyloxypropyl trimethoxysilane, 2-(meth)acryloyloxyethyl isocyanate, 1,1-bis(acryloyloxy)ethyl isocyanate, 2-(2-(meth)acryloyloxy ethyloxy)ethyl isocyanate, 3-(meth)acryloyloxypropyl triethoxysilane, and derivatives thereof.

Examples of compounds having one or more (meth)acryloylamino group per molecule include, but are not limited to, 4-(meth)acryloyl morpholine, N,N-dimethyl (meth)acrylamide, N,N-diethyl (meth)acrylamide, N-methyl (meth)acrylamide, N-ethyl (meth)acrylamide, N-propyl (meth)acrylamide, N-isopropyl (meth)acrylamide, N-butyl (meth)acrylamide, N-n-butoxymethyl (meth)acrylamide, N-hexyl (meth)acrylamide, N-octyl (meth)acrylamide, and derivatives thereof.

Examples of compounds having one or more vinyl ether groups per molecule include, but are not limited to, 3,3-bis(vinyloxymethyl)oxetane, 2-hydroxyethyl vinyl ether, 3-hydroxypropyl vinyl ether, 2-hydroxypropyl vinyl ether, 2-hydroxyisopropyl vinyl ether, 4-hydroxybutyl vinyl ether, 3-hydroxybutyl vinyl ether, 2-hydroxybutyl vinyl ether, 3-hydroxyisobutyl vinyl ether, 2-hydroxyisobutyl vinyl ether, 1-methyl-3-hydroxypropyl vinyl ether, 1-methyl-2-hydroxypropyl vinyl ether, 1-hydroxymethylpropyl vinyl ether, 4-hydroxycyclohexyl vinyl ether, 1,6-hexanediol monovinyl ether, 1,4-cyclohexane dimethanol monovinyl ether, 1,3-cyclohexane dimethanol monovinyl ether, 1,2-cyclohexane dimethanol monovinyl ether, p-xylene glycol monovinyl ether, m-xylene glycol monovinyl ether, o-xylene glycol monovinyl ether, diethylene glycol monovinyl ether, triethylene glycol monovinyl ether, tetraethylene glycol monovinyl ether, pentaethylene glycol monovinyl ether, oligoethylene glycol monovinyl ether, polyethylene glycol monovinyl ether, dipropylene glycol monovinyl ether, tripropylene glycol monovinyl ether, tetrapropylene glycol monovinyl ether, pentapropylene glycol monovinyl ether, oligopropylene glycol monovinyl ether, polypropylene glycol monovinyl ether, and derivatives thereof.

Examples of compounds having one or more vinyl aryl groups per molecule include, but are not limited to, styrene, divinylbenzene, methoxystyrene, ethoxystyrene, hydroxystyrene, vinyl naphthalene, vinyl anthracene, 4-vinylphenyl acetate, (4-vinylphenyl)dihydroxyborane, N-(4-vinylphenyl)maleimide, and derivatives thereof.

Examples of compounds having one or more vinyloxycarbonyl group per molecule include, but are not limited to, isopropenyl formate, isopropenyl acetate, isopropenyl propionate, isopropenyl butyrate, isopropenyl isobutyrate, isopropenyl caproate, isopropenyl valerate, isopropenyl isovalerate, isopropenyl lactate, vinyl acetate, vinyl propionate, vinyl butyrate, vinyl caproate, vinyl caprylate, vinyl laurate, vinyl myristate, vinyl palmitate, vinyl stearate, vinyl cyclohexane carboxylate, vinyl pivalate, vinyl octylate, vinyl monochloroacetate, divinyl adipate, vinyl acrylate, vinyl methacrylate, vinyl crotonate, vinyl sorbate, vinyl benzoate, vinyl cinnamate, and derivatives thereof.

Cationically Polymerizable Monomers

Examples of cationically polymerizable monomers include compounds having, per molecule, one or more cationic polymerizable groups, such as an epoxy ring (oxiranyl group), vinyl ether group, vinyl aryl group, and oxetanyl group.

Examples of compounds having one or more epoxy rings per molecule include, but are not limited to, glycidyl methyl ether, bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolac resin, hydrogenated bisphenol A diglycidyl ether, hydrogenated bisphenol F diglycidyl ether, hydrogenated bisphenol S diglycidyl ether,
3,4-epoxycyclohexylmethyl(3,4-epoxy)cyclohexane carboxylate, 2-(3,4-epoxycyclohexyl-5,5-spiro-3,4-epoxy)cyclohexane-meta-dioxane, bis (3,4-epoxycyclohexylmethyl)adipate, bis(3,4-epoxy-6-methylcyclohexylmethyl)adipate, 3,4-epoxy-6-methylcyclohexyl-3',4'-epoxy-6'-methylcyclohexane carboxylate, methylene bis(3,4-epoxycyclohexane), dicyclopentadiene diepoxide, di(3,4-epoxycyclohexylmethyl)ether of ethylene glycol, ethylene bis(3,4-epoxycyclohexane carboxylate), dioctyl epoxy hexahydrophthalate, di-2-ethylhexyl epoxy hexahydrophthalate, 1,4-butanediol diglycidyl ether, 1,6-hexanediol diglycidyl ether, glycerin triglycidyl ether, trimethylolpropane triglycidyl ether, polyethylene glycol diglycidyl ether, polypropylene glycol diglycidyl ether; polyglycidyl ethers of polyether polyols obtained by adding one or more alkylene oxides to an aliphatic polyhydric alcohol such as ethylene glycol, propylene glycol and glycerin; diglycidyl esters of aliphatic long chain dibasic acids; monoglycidyl ethers of aliphatic higher alcohols; monoglycidyl ethers of phenol, cresol, butyl phenol, or of polyether alcohols obtained by adding an alkylene oxide to these; and glycidyl esters of higher fatty acids.

Examples of compounds having one or more vinyl ether groups per molecule and of compounds having one or more vinyl aryl groups per molecule include the same compounds as those compounds exemplified as radically polymerizable compounds (a-2).

Examples of compounds having one or more oxetanyl groups per molecule include, but are not limited to, trimethylene oxide, 3,3-bis(vinyloxymethyl)oxetane, 3-ethyl-3-hydroxymethyl oxetane, 3-ethyl-3-(2-ethylhexyloxymethyl)oxetane, 3-ethyl-3-(hydroxymethyl)oxetane, 3-ethyl-3-[(phenoxy)methyl]oxetane, 3-ethyl-3-(hexyloxymethyl)oxetane, 3-ethyl-3-(chloromethyl)oxetane, 3,3-bis(chloromethyl)oxetane, 1,4-bis[(3-ethyl-3-oxetanylmethoxy)methyl]benzene, bis {[1-ethyl(3-oxetanyl)]methyl} ether, 4,4'-bis[(3-ethyl-3-oxetanyl)methoxymethyl]bicyclohexyl, 1,4-bis[(3-ethyl-3-oxetanyl)methoxymethyl]cyclohexane, and 3-ethyl-3{[(3-ethyloxetan-3-yl)methoxy]methyl}oxetane.

Oligomers

Examples of oligomers of the radically polymerizable monomer and the cationically polymerizable monomer include monofunctional or polyfunctional (meth)acrylic-based oligomers. One type or a combination of two or more types can be used.

Examples of the monofunctional or multifunctional (meth)acrylic-based oligomers include urethane (meth)acrylate oligomers, epoxy (meth)acrylate oligomers, polyether (meth)acrylate oligomers, and polyester (meth)acrylate oligomers.

Examples of urethane (meth)acrylate oligomers include polycarbonate-based urethane (meth)acrylate, polyester-based urethane (meth)acrylate, polyether-based urethane (meth)acrylate, and caprolactone-based urethane (meth)acrylate.

The urethane (meth)acrylate oligomer can be obtained through a reaction between a (meth)acrylate monomer having a hydroxyl group, and an isocyanate compound obtained by reacting a polyol with diisocyanate. Examples of the polyol include polycarbonate diols, polyester polyols, polyether polyols, and polycaprolactone polyols.

The epoxy (meth)acrylate oligomer is obtained by, for example, an esterification reaction between acrylic acid and an oxirane ring of a low molecular weight bisphenol type epoxy resin or a novolac epoxy resin.

The polyether (meth)acrylate oligomer is obtained by obtaining a polyether oligomer having hydroxyl groups at both ends through a dehydration condensation reaction of a polyol, followed by subjecting the hydroxyl groups at both ends to esterification with acrylic acid.

The polyester (meth)acrylate oligomer is obtained, for example, by obtaining a polyester oligomer having hydroxyl groups at both ends through condensation of a polycarboxylic acid and a polyol, followed by subjecting the hydroxyl groups at both ends to esterification with acrylic acid.

The weight average molecular weight of the monofunctional or polyfunctional (meth)acrylic oligomer is preferably 100000 or less and particularly preferably from 500 to 50000.

When the monomer or oligomer described above is used, it is preferable to use from 0.01 to 10 parts by mass of a photopolymerization initiator per 100 parts by mass of the monomer or oligomer.

The synthetic resin adhesive and the rubber-based adhesive are not particularly limited, and known adhesives such as the following thermoplastic adhesives and thermosetting adhesives can be used.

Examples of thermoplastic adhesives include, but are not limited to, polyvinyl acetates, polyvinyl alcohols, polyvinyl formals, polyvinyl butyrals, acrylic adhesives, polyethylene, chlorinated polyethylene, ethylene-vinyl acetate copolymers, ethylene-vinyl alcohol copolymers, ethylene-ethyl acrylate copolymers, ethylene-acrylic acid copolymers, ionomers, chlorinated polypropylenes, polystyrenes, polyvinyl chlorides, plastisols, vinyl chloride-vinyl acetate copolymers, polyvinyl ethers, polyvinylpyrrolidone, polyamides, nylons, saturated amorphous polyesters, and cellulose derivatives.

Examples of thermosetting adhesives include, but are not limited to, urea resins, melamine resins, phenolic resins, resorcinol resins, epoxy resins, polyurethanes, and vinyl urethanes.

Examples of rubber-based adhesives include, but are not limited to, natural rubbers, synthetic polyisoprenes, polychloroprenes, nitrile rubbers, styrene-butadiene rubbers, styrene-butadiene-vinylpyridine terpolymers, polyisobutylene-butyl rubber, polysulfide rubbers, silicone RTV, rubber chlorides, rubber bromides, kraft rubbers, block copolymers, and liquid rubbers.

Step Illustrated in FIG. 4C

In the next step, the IC tag 40 is placed on the base section 31. The planar area (occupied region) of the IC tag 40 is preferably smaller than the planar area of the base section 31. As a result, the base section 31 extends around the IC tag 40.

The IC tag 40 is a known IC tag that has an integrated circuit, and can read and write information using a reader (R/W) wirelessly. The IC tag 40 has information processing and storage functions, and it is one type of compact electronic device that works in response to wireless radio waves. When the IC tag 40 is attached to the tweezers 20, the tweezers 20 can be identified without contacting the tweezers 20, and therefore management of the usage history and loss prevention are facilitated.

In the step illustrated in FIG. 4B, when the base section 31 is not formed or the area of the base section 31 is formed to be smaller than the area of the IC tag 40, the IC tag 40 is placed such that the entire IC tag 40 or a portion thereof is in contact with the roughened section 22 of the tweezers 20. In this manner, when the IC tag 40 is placed in a state of being in contact with the tweezers 20, the tweezers 20 as such can function as an antenna for the IC tag 40, and therefore the IC tag 40 can be adopted even for a case of a very small IC tag with a low reception capability.

Step Illustrated in FIG. 4D

In the next step, a synthetic resin in a molten state or a solution state is deposited onto the base section 31 and the IC tag 40 to form a covering section 32 of the synthetic resin covering the base section 31 of synthetic resin and the IC tag 40, thereby encapsulating the IC tag 40 inside a synthetic resin part 30. In the step illustrated in FIG. 4B, even when the base section 31 is not formed, or when the area of the base section 31 is formed to be smaller than the area of the IC tag 40, a synthetic resin in a molten state or a solution state is deposited onto the IC tag 40 placed on the roughened section 22 to form the covering section 32 of the synthetic resin, thereby the IC tag 40 is encapsulated inside the synthetic resin part 30 including the covering section 32.

The amount of the synthetic resin that forms the covering section 32 is greater than the amount of the synthetic resin that forms the base section 31, and the amount of the synthetic resin that forms the covering section 32 can be, for example, approximately 5 to 15 times the amount of the synthetic resin that forms the base section 31. The height of the synthetic resin part 30 from the surface 21 of the tweezers 20 is preferably from 2 to 20 mm and more preferably from 3 to 10 mm.

The synthetic resin part 30 including the base section 31 and the covering section 32 formed by deposition of the synthetic resin can be left as is to be solidified or can be cured by heating, depending on the type of the synthetic resin used.

When an energy ray curable resin is used as the synthetic resin, the base section 31 and the covering section 32 are irradiated with energy rays (ultraviolet light, electron beams, etc.) and cured. The method of irradiation with energy rays is not particularly limited and may be performed until the uncured monomer or oligomer is cured. Furthermore, depending on the type of energy rays, the irradiation can be performed in a tightly sealed atmosphere.

As illustrated in FIG. 3 and FIG. 4D, in the medical apparatus made of a metal (tweezers) 20 having the IC tag 40 encapsulated inside the synthetic resin part 30, the base section 31 and the roughened section 22 formed on a portion of the surface 21 of the tweezers 20 are firmly adhered with high strength, and the base section 31 and the covering section 32 are integrated.

In the IC tag-equipped medical apparatus of an embodiment of the present invention, the IC tag 40 is encapsulated inside the synthetic resin part 30 including the base section 31 and the covering section 32, and the synthetic resin part 30 is firmly adhered to the medical apparatus with high strength, and therefore even if the medical apparatus is repeatedly used in surgeries and the like, and then repeatedly cleaned and sterilized after use, the IC tag 40 will not detach from the medical apparatus.

In addition, even when, in the step illustrated in FIG. 4B, the base section 31 is not formed or the base section 31 is formed with an area that is smaller than the area of the IC tag 40, the covering section 32 and the roughened section 12 are firmly adhered with high strength, and therefore, similarly, the IC tag 40 does not detach from the medical apparatus.

Thus, the IC tag-equipped medical apparatus of an embodiment of the present invention facilitates the identification of various surgical equipment and materials such as scalpels, scissors, forceps, and tweezers used in surgeries and the like, makes it possible to reliably and easily manage the usage history thereof, and can completely prevent their loss during surgeries and the occurrence of situations in which the loss itself is not noticed.

Examples

Example 1

In Example 1, a predetermined portion of a tweezers 20 like that illustrated in FIG. 2 was irradiated with laser under the following conditions to roughen the surface (FIG. 4A). The tweezers 20 was made of stainless steel.

Laser Irradiation Conditions

Oscillator: YLR-300-AC (single mode fiber laser) available from IPG Photonics Corporation
Light Focusing System: fc = 80 mm/fθ = 100 mm
Defocus distance: ±0 mm (constant)
Output power (W): 300
Wavelength (nm): 1070
Spot diameter (μm): 16
Energy density (MW/cm$^2$): $300/(\pi \times [0.0016 \text{ cm}/2]^2)$ = approximately 150 MW/cm$^2$
Laser irradiation rate (mm/sec): 10000
Irradiation pattern: bi-directional
Number of repetitions: 15
Number of lines: 120
Line interval (mm): 0.05
Processing time (s): 3.37

Hole Depth

The depth of the holes was determined by selecting a portion (area of 1 mm × 1 mm = 1 mm$^2$) of the surface (wide range of 6 × 10 = 60 mm$^2$) after laser beam irradiation, and then measuring the depth with the digital microscope M205C (Leica Microsystems GmbH). Specifically, five lines were drawn in parallel at intervals of 100 μm in a 1 mm × 1 mm square, and the depth was measured from observations of cross-section along the line portions. The average of the maximum depth measured at the five portions was taken as the depth of the holes (grooves).

The depth of the holes in the roughened section 22 of the surface 21 of the tweezers 20 used in Example 1 after roughening was 170 μm.

Next, a UV curable resin (liquid at normal temperature) was drawn by a small spuit, and one drop (approximately 0.005 ml) was dripped onto the roughened section 22 to cover the entire roughened section 22 and form the base section 31 (FIG. 4B).

Subsequently, the base section 31 was passed through a UV irradiation zone equipped with a high-pressure mercury-vapor lamp in an upper part while being moved in one direction at 1 m/minute. While passing the base section 31 through the UV irradiation zone, adjustments were made in order to apply a total of 0.3 J/cm$^2$ of energy to the UV curable resin of the base section 31.

As the UV curable resin, 100 parts by mass of urethane acrylate (trade name EBECRYL 8402, available from Daicel-Allnex Ltd.) and 5 parts by mass of a photoinitiator (Irgacure 1173, available from BASF) were used.

Next, an IC tag 40 (8 mm long × 3 mm wide × 2 mm thick) was picked up with a work tweezers and placed on the base section 31 (FIG. 4C). At this time, the base section 31 was slightly recessed.

Next, the same UV curable resin as that used in the base section 31 was drawn by a small spuit, and three drops (approximately 0.06 ml) were dripped onto the base section 31 and the IC tag 40, and a covering section 32 that covers both the base section 31 and the IC tag 40 was formed (FIG. 4D).

Next, the tweezers 20 were placed with the surface 21 oriented upward, and then irradiated with ultraviolet light from above using the high-pressure mercury-vapor lamp. Adjustments were made to apply a total of 3 J/cm$^2$ of energy to the UV curable resin of the synthetic resin part 30 on the tweezers 20.

In this manner, the tweezers 20 with the IC tag 40 encapsulated inside the synthetic resin part 30 was obtained. The synthetic resin part 30 was firmly adhered to the surface of the tweezers 20, and did not peel away or become damaged even when pressed strongly with a metal spatula (made of stainless steel) having a width of 10 mm.

Examples 2 to 5

Tweezers 20 having a synthetic resin part 30 (IC tag 40) were manufactured in the same manner as in Example 1 using a combination of 100 parts by mass of various energy curable resins and 5 parts by mass of a photoinitiator as described below.

Example 2: Epoxy acrylate (trade name EBECRYL 3708, available from Daicel-Allnex Ltd.), photoinitiator (Irgacure 1173, available from BASF)
Example 3: Acrylic monomer (trade name IRR214-K, available from Daicel-Allnex Ltd.), photoinitiator (Irgacure 1173, available from BASF)
Example 4: Alicyclic epoxy resin (trade name: Celloxide 2021P, available from Daicel Corporation), photoinitiator (CPI-101A, available from San-Apro Ltd.)
Example 5: Bisphenol A epoxy resin (trade name jER828, available from Mitsubishi Chemical Corporation), photoinitiator (CPI-101A, available from San-Apro Ltd.)

Each of the synthetic resin parts 30 in Examples 2 to 5 was firmly adhered to the surface of the tweezers 20, and none of the synthetic resin parts 30 peeled away or became damaged even when pressed strongly with a metal spatula (made of stainless steel) having a width of 10 mm.

Examples 6 to 8

A predetermined portion of the tweezers 20 as illustrated in FIG. 2 was irradiated with a pulsed wave laser beam to satisfy the conditions (a) to (f) shown in Table 1. The tweezers 20 were made of 64Ti.

[Table 1]

| | Example 6 | Example 7 | Example 8 |
|---|---|---|---|
| Metal plate type | 64Ti | | |

(continued)

|  |  | Example 6 | Example 7 | Example 8 |
|---|---|---|---|---|
| Thickness of metal plate (mm) | | 2.0 | | |
| Treated area (mm$^2$) | | 50 | | |
| Laser oscillator | | IPG-Yb fiber (YLP-RA-50-30-30) | | |
| Focusing optical system | | LXD30 + Hurry SCAN10 available from Scanlab GmbH (Beam expander 2X/ f$\theta$ 100 mm) | | |
| Output power (W) | | 30 | | |
| Wavelength (nm) | | 1069 | | |
| Spot diameter ($\mu$m) | | 48 | | |
| Frequency (KHz) | | 30 | | |
| Pulse width (nsec) | | 50 | | |
| Irradiation pattern | | FIG. 5 | FIG. 6 | FIG. 7 |
| (a) | Irradiation direction and angle | Angle of 90 degrees relative to metal plate | | |
| (b) | irradiation rate (mm/sec) | 250 | 500 | 800 |
| (c) | Energy density (GW/cm$^2$) | 1.106 | | |
| (d) | Number of repetitions (times) | 5 | 60 | 10 |
| (e) | Irradiation form | Irradiation in contact with steel plate | | |
| (f) | Line interval (mm) | 0.028 | 0.06 | - |
| Maximum Hole Depth ($\mu$m) | | 180 | 210 | 200 |

The details of the irradiation patterns listed in Table 1 were as follows.

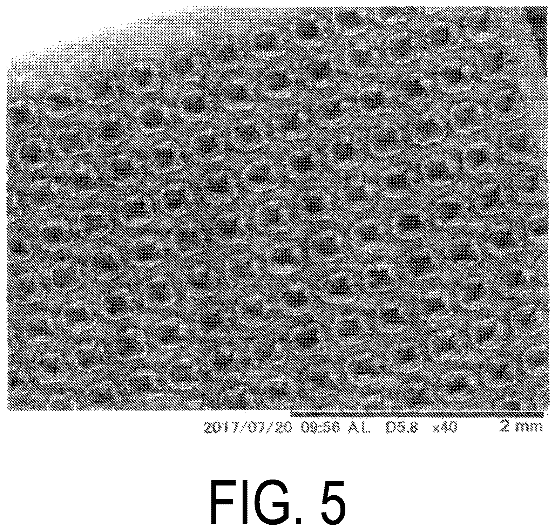

Square holes (FIG. 5): The irradiation was performed in a 150 $\mu$m straight line with a pulsed wave laser beam having a spot diameter described in Table 1, and then in the same manner in the opposite direction at an interval of 0.028 mm (distance between centers of adjacent grooves), and with five repetitions of this process considered to be a single operation, the same operation was repeated five times, and square holes having a maximum depth of 180 $\mu$m were formed. The same operation was further repeated to form a plurality of square holes with an interval between adjacent square holes of 150 $\mu$m.

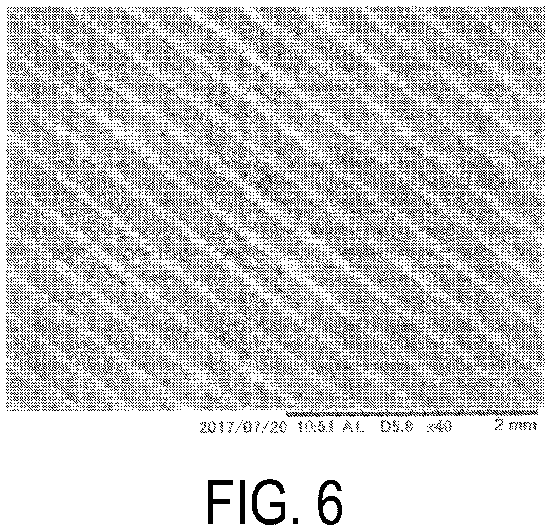

Meandering (FIG. 6): The irradiation was performed in a straight line with a pulsed wave laser beam in one direction, after which the course of the pulsed wave laser beam was reversed at an interval of 0.06 mm, and irradiation with the pulsed wave laser beam was similarly performed in a straight line in the opposite direction, thereby completing a round trip, and this round trip process was repeated twice to form a single groove. Then, the next groove was formed with an interval of 0.105 mm therefrom, and with this process considered to be a single operation, the same operation was repeated 60 times.

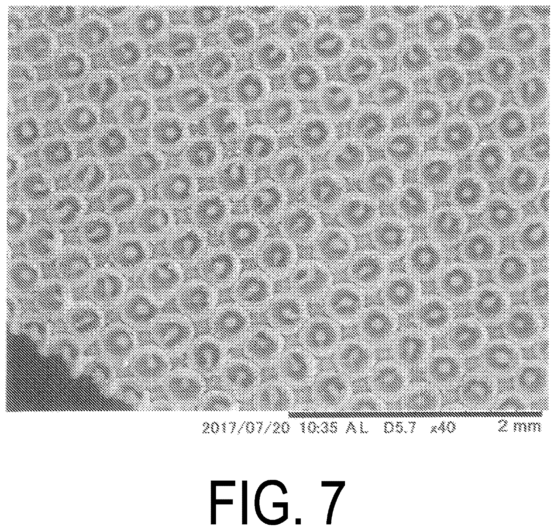

Circles (FIG. 7): A pulsed wave laser beam was scanned in a circular shape with a diameter of 200 $\mu$m for 10 repetitions using the spot diameter and irradiation rate described in Table 1, thereby a circle having a diameter of 200 $\mu$m or slightly greater was formed. The same operation was repeated to form a plurality of circles. The distance between centers of adjacent circles was 0.4 mm.

Surface image (SEM image) of titanium tweezers after irradiation with a laser beam are presented in FIGS. 5 to 7. As illustrated in FIGS. 5 to 7, the surfaces of the titanium tweezers were confirmed to have porous structures. The maximum depth was measured using a digital microscope VHX-6000 (available from Keyence Corporation).

Subsequently, tweezers 20 with the IC tag 40 encapsulated inside the synthetic resin part 30 were obtained in the same manner as in Example 1.

Industrial Applicability

The medical apparatus and manufacturing method thereof of an embodiment of the present invention can be applied to various types of medical apparatus made of a metal such as scalpels, scissors, forceps, and tweezers used in surgery or the like, and the medical apparatus of an embodiment of the present invention can be used as a medical apparatus with a history recording function and a loss prevention function.

Reference Signs List

10      Medical apparatus (forceps)

11      (21) Surface of medical apparatus

12      (22) Roughened section

20      Medical apparatus (tweezers)

30      Synthetic resin part

31      Base section (lower layer part)

32      Covering section (upper layer part)

40      IC Tag

**Claims**

1.  A medical apparatus made of a metal and having an IC tag encapsulated, the medical apparatus comprising:

    a synthetic resin part affixed to a roughened section of the medical apparatus made of a metal, the roughened section having a porous structure including holes with a depth from 10 to 900 $\mu$m; and
    the IC tag encapsulated inside the synthetic resin part.

2.  The medical apparatus according to claim 1, wherein the medical apparatus is a scalpel, scissors, forceps, or tweezers.

3.  The medical apparatus according to claim 1, wherein the entire IC tag or a portion of the IC tag is in contact with the roughened section of the medical apparatus.

4.  A method for manufacturing a medical apparatus, the method comprising:

    (A) irradiating a portion of a surface of a medical apparatus made of a metal with a laser beam to roughen the surface and form a roughened section;
    (B) depositing a synthetic resin in a molten state or a solution state onto a portion of the medical apparatus including the roughened section to form a base section of the synthetic resin;
    (C) placing an IC tag on the base section; and
    (D) depositing a synthetic resin in a molten state or a solution state onto the base section and the IC tag to form a covering section of the synthetic resin that covers the base section of the synthetic resin and the IC tag, thereby encapsulating the IC tag inside a synthetic resin part including the base section and the covering section.

5.  The method for manufacturing a medical apparatus according to claim 4, wherein a planar area of the IC tag is smaller than a planar area of the base section.

6.  A method for manufacturing a medical apparatus, the method comprising:

    (A) irradiating a portion of a surface of a medical apparatus made of a metal with a laser beam to roughen the surface and form a roughened section;
    (B) placing an IC tag on the roughened section; and

(C) depositing a synthetic resin in a molten state or a solution state onto the roughened section and the IC tag to form a covering section of the synthetic resin that covers the roughened section and the IC tag, thereby encapsulating the IC tag inside a synthetic resin part comprising the covering section.

7. The method for manufacturing a medical apparatus according to claim 4 or 6, wherein step (A) includes forming a porous structure having holes with a depth from 10 to 900 $\mu$m.

8. The method for manufacturing a medical apparatus according to claim 4 or 6, wherein the medical apparatus is a scalpel, scissors, forceps, or tweezers.

9. The method for manufacturing a medical apparatus according to claim 4 or 6, wherein step (A) includes performing irradiation with a continuous wave laser beam or a pulsed wave laser beam.

10. The method for manufacturing a medical apparatus according to claim 4 or 6, wherein step (A) includes performing continuous irradiation with a laser beam having an energy density of 1 MW/cm$^2$ or greater at an irradiation rate of 2000 mm/sec or greater using a laser device.

11. The method for manufacturing a medical apparatus according to claim 4 or 6, wherein step (A) includes performing irradiation with a pulsed wave laser beam with adjustment of one or more requirements selected from the following conditions (a) to (f):

(a) An irradiation direction and an irradiation angle when irradiating the medical apparatus made of a metal with the laser beam;
(b) An irradiation rate when irradiating the medical apparatus made of a metal with the laser beam;
(c) An energy density when irradiating the medical apparatus made of a metal with the laser beam;
(d) A number of repetitions when irradiating the medical apparatus made of a metal with the laser beam;
(e) An irradiation form when irradiating the medical apparatus made of a metal with the laser beam;
(f) An interval between lines or dots when irradiating the medical apparatus made of a metal with the laser beam.

# FIG. 1

FIG. 2

# FIG. 3

# FIG. 4A

21 — 22 ↓ 20

# FIG. 4B

21 — 31 20

# FIG. 4C

40 31 21 — 20

# FIG. 4D

30 21 — 20

2017/07/20 09:56 AL. D5.8 x40    2 mm

FIG. 5

2017/07/20 10:51 AL. D5.8 x40 2 mm

# FIG. 6

2017/07/20 10:35 AL  D5.7  x40      2 mm

# FIG. 7

## INTERNATIONAL SEARCH REPORT

| International application No. |
| --- |
| PCT/JP2018/012869 |

**A. CLASSIFICATION OF SUBJECT MATTER**
Int. Cl. A61B90/90(2016.01)i, A61B17/28(2006.01)i, A61B17/30(2006.01)i, A61B17/3211(2006.01)i

According to International Patent Classification (IPC) or to both national classification and IPC

**B. FIELDS SEARCHED**

Minimum documentation searched (classification system followed by classification symbols)
Int. Cl. A61B90/90, A61B17/28, A61B17/30, A61B17/3211

Documentation searched other than minimum documentation to the extent that such documents are included in the fields searched
Published examined utility model applications of Japan    1922-1996
Published unexamined utility model applications of Japan    1971-2018
Registered utility model specifications of Japan    1996-2018
Published registered utility model applications of Japan    1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2014/017530 A1 (JAPAN INFORMATION SYSTEM CO., LTD.) 30 January 2014, paragraphs [0038]-[0040] (Family: none) | 1-2, 4-5, 7-11 |
| Y | US 2016/0296299 A1 (CARETAG SURGICAL APS) 13 October 2016, claims 1, 19, paragraphs [0131]-[0133], [0219], fig. 6 & WO 2015/086775 A1 | 1-3, 6-11 |
| Y | JP 2017-019189 A (DAICEL POLYMER LTD.) 26 January 2017, paragraphs [0011]-[0015] (Family: none) | 1-11 |
| Y | JP 2014-117724 A (POLYPLASTICS CO., LTD.) 30 June 2014, paragraphs [0029]-[0033] & CN 103862164 A | 1-11 |

☒ Further documents are listed in the continuation of Box C.    ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 24.05.2018 | 05.06.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

| International application No. |
| --- |
| PCT/JP2018/012869 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | WO 2016/129392 A1 (OMRON CORP.) 18 August 2016, paragraphs [0034]-[0054] & JP 2016-144823 A | 1-11 |
| A | US 2016/0128799 A1 (LM-INSTRUMENTS OY) 12 May 2016, paragraphs [0026]-[0041], fig. 1a-2c & JP 2016-518212 A, paragraphs [0017]-[0030], fig. 1, 2 & WO 2014/184441 A1 & EP 2803330 A1 | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014017530 A
- JP 5774246 B
- JP 5701414 B
- JP 5860190 B
- JP 5890054 B
- JP 5959689 B
- JP 2016043413 A
- JP 2016036884 A
- JP 2016044337 A
- JP 5848104 B
- JP 5788836 B
- JP 5798534 B
- JP 5798535 B
- JP 2016203643 A
- JP 2016078090 A
- JP 2016124024 A